# EUROPEAN PATENT APPLICATION

(11) **EP 2 857 028 A1**
(43) Date of publication of application: **08.04.2015**
(21) Application number: 13793133.3
(22) Date of filing: 17.05.2013
(51) Int. Cl.: A61K 36/18, A23L 1/30, A23L 1/305, A61K 31/375, A61K 31/7048, A61K 35/20, A61K 38/00, A61P 19/08, A61P 19/10, A61P 43/00

(54) **AGENT CONTAINING ASCORBIC ACID DERIVATIVE, AND USE FOR SAID AGENT**

(30) Priority: 25.05.2012 JP 2012119960; 25.05.2012 JP 2012119961; 25.05.2012 JP 2012120006
(71) Applicant: LION CORPORATION, Tokyo 130-8644 (JP)
(72) Inventor: TANAKA, Shoko, Tokyo 130-8644 (JP); KOIKE, Yasushi, Tokyo 130-8644 (JP); KATAOKA, Shinsuke, Tokyo 130-8644 (JP)
(74) Representative: Hoffmann Eitle
(86) International application number: PCT/JP2013/063800
(87) International publication number: WO 2013/176054

(57) **Abstract**

An object of the present invention is to provide an active ingredient that can remarkably exhibit an effect of preventing and/or relieving osteoporosis or an effect of promoting bone formation and is usable as a food or drink product. The present invention provides an agent for preventing and/or relieving osteoporosis comprising component (A): an ascorbic acid derivative as an active ingredient, a composition comprising the agent, an composition for oral administration comprising component (A) and component (B): an extract from a plant in the family Piperaceae as active ingredients, a composition for promoting bone formation containing component (A) and component (D): sorbitol and/or a derivative thereof as active ingredients, and a composition, such as a pharmaceutical product, a quasi-pharmaceutical product, and a food or drink product, comprising any of these compositions.

## Description

### Field of the Invention

The present invention relates to an agent comprising an ascorbic acid derivative and the use thereof.

### Background of the Invention

In recent years, scientific progress and the increased standard of living have extended the lifespan of the human. As the elderly population increases, the incidence of osteoporosis increases steadily year after year. The current number of osteoporotic patients is reported to be 4 to 5 million. Among osteoporosis, postmenopausal osteoporosis, which is categorized as primary osteoporosis, occurs in postmenopausal women at the age of 50 and older at a high rate and accounts for 90% or more of all the osteoporotic patients. Based on these, osteoporosis is taken seriously in modern society as a disease comparable to lifestyle-related diseases typified by diabetes. In addition, a prolonged bedridden state due to lumbar vertebral and/or femoral fractures caused by osteoporosis presents the risk of inducing dementia. Hence for the purpose of overall improvement in Quality of life (QOL), prompt development of drugs and food for preventing and/or treating osteoporosis has been desired.

Osteoporosis is a systemic bone disease that decreases bone mass and degenerates the fine structure of bone tissue due to a decrease in bone (mainly cancellous bone)-forming components such as calcium and collagen. Osteoporosis is characteristically accompanied by pain in the bone, embrittlement of the bone, and the risk of fractures. Development of osteoporosis is believed to be involved in many factors directly or indirectly and multifactorially. Such factors that have been reported include decreased secretion of estrogen due to the menopause, nutritional factors such as calcium metabolism-regulating hormones and calcium intake, and mechanical stress such as moderate exercises and gravity. The complex interrelationships of these factors are believed to affect the metabolism of the bone.

Human bone is continually being resorbed and formed, and cells playing a key role in the metabolism of the bone are osteoblasts responsible for bone formation and osteoclasts responsible for bone resorption. Osteoblasts have been confirmed to decrease in number along with aging. Growth, preservation, and repair of bone tissue rely on the balance between the rate of bone formation and the rate of bone resorption. When this balance is disturbed and calcification ability is impaired, bone resorption outpaces bone formation to lead to a decrease in the bone mass, causing diseases such as osteoporosis.

As drugs for osteoporosis, calcium, activated vitamin D3, estrogen, calcitonin, ipriflavone, vitamin K2, and bisphosphonate-related compounds are used. As food to strengthen the bone, calcium or vitamin D is primarily used at present and recently isoflavonoids such as genistin are to be used.

L-ascorbic acid is known to be important for synthesis of collagen in a living organism, which is a main component of the bone matrix. In other words, L-ascorbic acid is essential in biosynthesis of amino acids that are present specifically in collagen, such as hydroxyproline and hydroxylysine, and is known to promote collagen synthesis and also promote osteoblast differentiation and bone formation when added to an osteoblast culture system, for example.

L-ascorbic acid has a disadvantage that it is subject to oxidative decomposition and therefore loses its physiological activity easily. In order to stabilize L-ascorbic acid, use of L-ascorbic acid derivatives such as sugar derivatives and ester derivatives is suggested. Non-patent Document 1 describes that L-ascorbic acid phosphate is more stable than L-ascorbic acid and L-ascorbic acid 2-phosphate has an effect of promoting growth and differentiation of cultured osteoblasts.

Plants in the family Piperaceae exhibit hotness, spiciness, and tastiness *(umami)* and are therefore used as spice. Extracts of Piper longum L. in the family Piperaceae are known to exhibit actions including blood circulation-promoting action and vasodilating action in living organisms. Extracts of Piper longum L. are also known to have an effect of relieving sensitivity to the cold based on their heating efficacy (Patent Document 1) and an effect of relieving swelling based on their blood circulation-promoting action (Patent Document 2). A composition containing Piper longum L. for relieving menopause fatigue is also known (Patent Document 3).

Sorbitol is a sugar alcohol and is used as an additive, such as a sweetener, a humectant, and a thickener, in food products, cosmetics, and pharmaceutical products.

### Citation List

### Patent Literature

Patent Literature 1: Japanese Patent Application Laid-open No. 2003-40788
Patent Literature 2: Japanese Patent Application Laid-open No. 2006-104109
Patent Literature 3: Japanese Patent Application Laid-open No. 2011-73973
Non Patent Literature 1: Cell Biol Int. 2004;28(4):255-65.

### Summary of the Invention

### Problem to be solved by the Invention

Any of these drugs for osteoporosis, however, does not give a satisfactory therapeutic effect. In particular, a calcium agent is required to be taken in a very large amount to be effective. In addition, their side effects present a problem as explained in the following. Calcitonin tends to trigger drug resistance and cannot be administered orally. Activated vitamin D3 tends to cause hypercalcemia. Bisphosphonate-related compounds inhibit bone formation. As for estrogen preparations, prolonged administration thereof for 6 months or longer is frequently accompanied by side effects such as facial flush, mammalgia, and abnormal bleeding from a genital, namely uterus or vagina. In addition, these drugs are not used for preventing osteoporosis but are supposed to be administered after the diagnosis of osteoporosis. Besides, purchase of drugs for osteoporosis results in high medical expenses of the patients.

Conventional food to strengthen the bone has exhibited neither an effect of preventing osteoporosis nor an adequate effect of promoting bone formation. If the food is made to prevent osteoporosis and promote bone formation, a large amount of an active ingredient is required to be added alone to the food, which is not suitable for an ingredient of the food and presents a problem of impaired palatability of the food.

Involvement of a plant in the family Piperaceae or an extract thereof in bone formation and other metabolism of the bone has not been known to date.

Involvement of sorbitol in bone formation has not been known to date, either.

An object of the present invention is to provide an agent that can be administered orally, can remarkably exhibit an effect of preventing and/or relieving osteoporosis or an effect of promoting bone formation, and is usable as a food or drink product.

### Solution to Problem

The inventors of the present invention experimentally administered an L-ascorbic acid derivative to an animal thereby finding that the administration remarkably increases a cancellous bone density. From this finding, they reached a conclusion that an agent comprising an L-ascorbic acid derivative is useful as an active ingredient of a drug for preventing and/or relieving osteoporosis. The inventors of the present invention have also found that a combination of an L-ascorbic acid derivative and an extract of a plant in the family Piperaceae enables significant improvement of bone formation. The inventors of the present invention have further found that a combination of an L-ascorbic acid derivative and sorbitol or a derivative thereof enables significant improvement of bone formation. The present invention is based on these findings.

The invention provides the following.
[1] An agent for preventing and/or relieving osteoporosis, the agent comprising component (A): an ascorbic acid derivative as an active ingredient.
[2] The agent according to the above [1], wherein component (A) is ascorbic acid phosphate magnesium salt and/or ascorbic acid 2-glucoside.
[3] A composition for preventing and/or relieving osteoporosis, the composition comprising component (A): an ascorbic acid derivative and a pharmacologically acceptable carrier.
[4] A composition for oral administration, comprising component (A): an ascorbic acid derivative and component (B): an extract from a plant in the family Piperaceae as active ingredients.
[5] The composition according to the above [4], wherein component (B) is an extract of Piper longum L.
[6] The composition according to the above [4] or [5], wherein component (A) is one or more ascorbic acid derivatives selected from sodium ascorbate, ascorbic acid phosphate magnesium salt, and ascorbic acid 2-glucoside.
[7] The composition according to any one of the above [4] to [6], further comprising component (C): lactoferrin.
[8] The composition according to the above [7], wherein component (C) is lactoferrin derived from cow milk.
[9] The composition according to any one of the above [4] to [8], wherein the composition promotes bone formation or prevents and/or relieves osteoporosis.
[10] A composition for promoting bone formation, the composition comprising component (A): an ascorbic acid derivative and component (D): sorbitol and/or a sorbitol derivative as active ingredients.
[11] The composition according to the above [10], wherein the composition prevents and/or relieves osteoporosis.
[12] The composition according to any one of the above [3] to [11], further comprising a pharmacologically acceptable carrier.
[13] The composition according to any one of the above [3] to [12], wherein the composition is a pharmaceutical product, a quasi-pharmaceutical product, or a food or drink product.

The present invention also provides [14] to [19] below.
[14] A method for preventing and/or relieving osteoporosis, comprising the step of administrating component (A): an ascorbic acid derivative.
[15] Use of component (A): an ascorbic acid derivative for the production of an agent for preventing and/or relieving osteoporosis.
[16] A method for promoting bone formation, comprising administrating component (A): an ascorbic acid derivative and component (B): an extract from a plant in the family Piperaceae.
[17] Use of component (A): an ascorbic acid derivative and component (B): an extract from a plant in the family Piperaceae for the production of a composition for promoting bone formation.
[18] A method for promoting bone formation, comprising administrating component (A): an ascorbic acid derivative and component (D): sorbitol and/or a derivative thereof.
[19] Use of component (A): an ascorbic acid derivative and component (D): sorbitol and/or a derivative thereof for the production of a composition for promoting bone formation.

The following inventions are provided as a first embodiment of the present invention.
[1-1] An agent for preventing and/or relieving osteoporosis, comprising an ascorbic acid derivative as an active ingredient.
[1-2] The agent for preventing and/or relieving osteoporosis according to the above [1-1], wherein the ascorbic acid derivative is ascorbyl phosphate magnesium salt and/or ascorbyl 2-glucoside.
[1-3] A food or drink product, comprising the agent for preventing and/or relieving osteoporosis according to the above [1-1] or [1-2].

The following inventions are provided as a second embodiment of the present invention.
[2-1] An composition for oral administration, comprising component (A): an ascorbic acid derivative and component (B): an extract from a plant in the family Piperaceae as active ingredients.
[2-2] The composition for oral administration according to the above [2-1], wherein component (B) is an extract of Piper longum L.
[2-3] The composition for oral administration according to the above [2-1] or [2-2], wherein component (A) is one or more ascorbic acid derivatives selected from sodium ascorbate, ascorbic acid phosphate magnesium salt, and ascorbic acid 2-glucoside.
[2-4] The composition for oral administration according to any one of the above [2-1] to [2-3], further comprising component (C): lactoferrin.
[2-5] The composition for oral administration according to the above [2-4], wherein component (C) is lactoferrin derived from cow milk.
[2-6] A composition for promoting bone formation, comprising component (A): an ascorbic acid derivative and component (B): an extract from a plant in the family Piperaceae as active ingredients.
[2-7] The composition for promoting bone formation according to the above [2-1] or [2-2], wherein the composition prevents and/or relieves osteoporosis.
[2-8] A food or drink product, comprising the composition for oral administration according to any one of the above [2-1] to [2-5] or the composition for promoting bone formation according to [2-6] or [2-7].

The following inventions are provided as a third embodiment of the present invention.
[3-1] An agent for promoting bone formation, comprising component (A): an ascorbic acid derivative and component (D): sorbitol and/or a derivative thereof.
[3-2] The agent for promoting bone formation according to the above [3-1], wherein the agent prevents and/or relieves osteoporosis.
[3-3] A composition for promoting bone formation, comprising the agent for promoting bone formation according to the above [3-1] or [3-2].
[3-4] A food or drink product, comprising the agent for promoting bone formation according to the above [3-1] or [3-2] or the composition for promoting bone formation according to the above [3-3].

### Advantageous Effects of the Invention

The present invention provides an agent that can prevent osteoporosis and/or fractures. The present invention can also remarkably promote bone formation and therefore provides a composition that can prevent osteoporosis and/or fractures. Hence the present invention can reduce the risk of osteoporosis and/or fractures and, after the development of osteoporosis, can relieve the symptom.

### Brief Description of Drawings

FIG. 1 is a graph depicting the volume of voids in cancellous bone in Example 1 (APM administered) and Comparative Example 1 (DW administered).
FIG. 2 is sectional views of cancellous bone in Example 1 (APM administered), Example 2 (A2G administered), and Comparative Example 1 (DW administered).
FIG. 3 is a sectional view of cancellous bone in Comparative Example 2 (healthy mouse).
FIG. 4 is a graph depicting BMD in Example 1 (APM administered), Comparative Example 1 (DW administered), and Comparative Example 2 (healthy mouse).
FIG. 5 is a graph depicting the result of expression rate of alkaline phosphatase gene in Examples 3 to 5, Comparative Examples 3 to 6, and Reference Example 1.
FIG. 6 is a graph depicting the result of expression rate of collagen gene in Examples 6 and 7, Comparative Examples 7 to 10, and Reference Example 2.
FIG. 7 is a graph depicting the result of expression rate of collagen gene in Examples 8 to 11, Comparative Examples 11 to 15, and Reference Example 3.
FIG. 8 is a graph depicting the result of expression rate of alkaline phosphatase gene in Examples 12 to 14 and Comparative Examples 16 to 19.

### Description of Embodiments

In the present invention, component (A) is an ascorbic acid derivative. The ascorbic acid derivative refers to a compound resulting from substitution of a part of ascorbic acid ((R)-3,4-dihydroxy-5-((S)-1,2-dihydroxyethyl)furan-2(5H)-one, vitamin C) with an atom or a substituent. Ascorbic acid from which the ascorbic acid derivative is derived may be any of a D form, an L form, and a DL form and is preferably an L form. The ascorbic acid derivative is not particularly limited provided that it is usable in the field of pharmaceutical products, quasi-pharmaceutical products, cosmetics, or food products. Examples of the ascorbic acid derivative may include ester derivatives of ascorbic acid or salts thereof, ether derivatives of ascorbic acid or salts thereof, and salts of ascorbic acid.

Examples of the ester derivatives of ascorbic acid may include esters of ascorbic acid with a carboxylic acid, sulfuric acid, a sulfonic acid, and a phosphoric acid. Examples of a moiety of ascorbic acid to be subjected to esterification may include hydroxy groups on the 2-, 3-, 5-, and 6-position of ascorbic acid. Each of the ester derivatives of ascorbic acid may have 1 or more esterified moieties on ascorbic acid, and 1 or more hydroxy groups selected from the hydroxy groups exemplified above may be subjected to esterification. When there are 2 or more esterified moieties, each esterified moiety may have the same or a different acid to form an ester.

Examples of the carboxylic acid may include aliphatic carboxylic acids and aromatic carboxylic acids.

Examples of the sulfonic acid may include sulfonic acids and alkylsulfonic acids. The alkylsulfonic acids are usually C₁₋₆ alkylsulfonic acids, and examples thereof may include methylsulfonic acid and ethylsulfonic acid.

Examples of the phosphoric acid may include phosphoric acid, phosphoric acid monoesters, and phosphoric acid diesters, and specific examples thereof may include the following compounds: phosphoric acid; mono-alkyl esters of phosphoric acid including a phosphoric acid monoester in which one hydrogen atom on phosphoric acid is substituted with an alkyl group selected from C₁₋₆ alkyl groups such as a methyl group, an ethyl group, a propyl group, an isopropyl group, a butyl group, a s-butyl group, a t-butyl group, an isobutyl group, a pentyl group, and a hexyl group; and di-alkyl esters of phosphoric acid including a di-alkyl ester of phosphoric acid in which two hydrogen atoms on phosphoric acid are substituted with two alkyl groups selected from C₁₋₆ alkyl groups such as a methyl group, an ethyl group, a propyl group, an isopropyl group, a butyl group, a s-butyl group, a t-butyl group, an isobutyl group, a pentyl group, and a hexyl group. The two alkyl groups in the above di-alkyl ester of phosphoric acid may be the same as or different from each other.

Examples of the ether derivatives of ascorbic acid may include ascorbic acid glucosides such as ascorbic acid 2-glucoside.

The ascorbic acid derivative may be a salt of ascorbic acid or a salt of an ascorbic acid derivative. Examples of the salt may include the following salts: various metal salts including alkali metal salts such as sodium and potassium, alkaline-earth metal salts such as magnesium, calcium and barium, and polyvalent metal salts such as aluminum; ammonium salts such as ammonium and tricyclohexylammonium; and various alkanolamine salts such as monoethanolamine, diethanolamine, triethanolamine, monoisopropanolamine, diisopropanolamine and triisopropanolamine.

In the present invention, the ascorbic acid derivatives used preferably are: salts of L-ascorbic acid such as sodium salts; phosphoric acid ester derivatives of L-ascorbic acid including L-ascorbic acid monophosphate sodium salt and magnesium L-ascorbyl phosphate such as ascorbic acid phosphate magnesium salt (APM), and salts thereof; ascorbic acid glucoside such as ascorbic acid 2-glucoside, and salts thereof; ascorbyl tetraisopalmitate (VCIP) and salts thereof; and ascorbic acid 2-phosphate 6-palmitate (APPS) and salts thereof. Ascorbic acid derivatives used more preferably are sodium L-ascorbate, magnesium L-ascorbyl phosphate, and ascorbic acid 2-glucoside such as ascorbic acid 2-0-α-glucoside (A2G).Ascorbic acid derivatives used further preferably are magnesium L-ascorbyl phosphate and ascorbic acid 2-glucoside such as ascorbic acid 2-0-α-glucoside (A2G).

The ascorbic acid derivatives used may be ones resulting from artificial synthesis such as chemical synthesis or commercially available products.

Component (A) may be a single ascorbic acid derivative or a combination of two or more ascorbic acid derivatives.

In the present invention, component (B) is an extract from a plant in the family Piperaceae.

Examples of the plant in the family Piperaceae may include plants in the genus such as the genus Piper, Arctottonia Trel., Macropiper Miq., Manekia Trel., the genus Peperomia (Peperomia Ruiz & Pav.), Pothomorphe Miq., Sarcorhachis Trel., Trianaeopiper Trel., Verhuellia Miq., and Zippelia Blume, and a plant in the genus Piper is preferable. Examples of the plant in the genus Piper may include Piper nigrum L., Piper longum L. (Long pepper), and Piper retrofractum Vahl. Piper longum L. is an evergreen vine belonging to the genus Piper in the family Piperaceae, mainly distributed in Southeast Asia.

The site of the plant subjected to extraction is not particularly limited, and may be the whole plant body or part of the plant body such as leaves, stems, vines, leaves, fruits, or seeds.

When a part of the plant body is used, the part separated from the plant body may be used as it is or may be dried in the sun, by a machine, or the like.

The method for extraction is not particularly limited, and examples thereof may include a method of extraction with a solvent and a method of extraction by supercritical extraction with carbon dioxide. As for the method of extraction with a solvent, examples of the extraction solvent may include water, alcohols such as methanol, ethanol, n-propanol, isopropanol, and butanol, polyhydric alcohols such as ethylene glycol and propylene glycol, acetone, methyl ethyl ketone, methyl butyl ketone, ethyl acetate, glycerol, acetic acid, and propionic acid. The extraction solvent may be used alone or as an optional combination of two or more solvents as a mixed solution. The extraction solvent is preferably water, an alcohol, a polyhydric alcohol, or a mixed solution thereof.

The conditions for the extraction method are not particularly limited and can be selected, as appropriate, depending on the extraction method, the type of the extraction feedstock, or the like. In the case of extraction with a solvent, the ratio of the extraction feedstock to the extraction solvent preferably satisfies the mass ratio of extraction feedstock: extraction solvent = about 1:2 to 1:50. The extraction temperature is preferably 5°C to 80°C. The duration of extraction is preferably 1 hour to 1 week. Extraction may be performed by immersing the extraction feedstock in the extraction feedstock and may be performed with stirring where appropriate, preferably with stirring. The pH during extraction may be selected, as appropriate, provided that it is not extremely acidic or alkaline.

The product resulting from extraction with a solvent may be used as it is as the extract from a plant in the family Piperaceae when the extraction solvent is a non-toxic solvent such as water, ethanol, and water/ethanol (hydrous ethanol). Alternatively, such a product may be diluted into a diluted solution or prepared into concentrated extract, dry powder by lyophilization or the like, or paste, to be used as the extract from a plant in the family Piperaceae. On the other hand, in the use of the solvent that may be toxic to living organisms, it is preferable to distil the solvent off and dilute the resulting dry matter with a non-toxic solvent before use.

Extracts from plants in the family Piperaceae are commercially available, and, in the present invention, commercially available extract products from plants in the family Piperaceae can also be used. Examples of commercially available extract products from plants in the family Piperaceae may include "Piper longum L. extract MF" (Maruzen Pharmaceuticals Co., Ltd.).

Component (B) may be a single extract from a plant in the family Piperaceae or a combination of two or more extracts from plants in the family Piperaceae. Component (B) may also be two or more extracts derived from the same plant in the family Piperaceae obtained with different extraction conditions, an extract resulting from single extraction from a combination of two or more plants in the family Piperaceae, or a combination of two or more extracts selected from such extracts.

In the present invention, component (C) is lactoferrin. Where the composition comprises component (C), the composition of the present invention can promptly exhibit an effect of promoting bone formation. The lactoferrin is :commercially available lactoferrin; lactoferrin separated by a conventional procedure such as ion exchange chromatography from colostrum, transitional milk, mature milk, late lactation milk, or the like from a mammal such as human, cow, sheep, goat, or horse, or processed product of such milk such as skim milk or whey; or lactoferrin produced from a plant such as tomato, rice or tobacco. A commercially available lactoferrin may be used, or a lactoferrin prepared by a known method may be used. The lactoferrin derived from cow milk is preferable.

Lactoferrin is commercially available, and, in the present invention, commercially available lactoferrin can be used.

Component (C) may be a single kind of lactoferrin or a combination of two or more kinds of lactoferrin.

In the present invention, component (D) is sorbitol and/or a derivative thereof. Sorbitol (glucitol) is a kind of sugar alcohol. The sorbitol is not particularly limited in its optical isomerism, may be a D form or an L form, and is usually a D form. The production method of sorbitol is not particularly limited, and may be chemically synthesized, or may be extracted from natural material.

Sorbitol is commercially available, and, in the present invention, commercially available sorbitol can be used. Examples of commercially available sorbitol may include "Sorbit KK-N" (manufactured by Kirin Kyowa Foods Co., Ltd.), "Sorbitol" (manufactured by Mitsubishi Shoji Foodtech Co., Ltd.), and "Sorbitol" (manufactured by B Food Science Co., Ltd.).

The sorbitol derivative refers to a compound resulting from substitution of a hydrogen atom bonded to a constituent carbon atom of sorbitol with a substituent such as an alkyl group other than a hydrogen atom, a salt of sorbitol, and a salt of the compound. Examples of the salt are the same as those exemplified as the salt of the ascorbic acid derivatives.

Component (D) may be a single kind of sorbitol or a derivative thereof, a combination of two or more kinds of sorbitol or derivatives thereof that are different in optical isomerism, a production method, and/or the like, or a combination of sorbitol and a derivative thereof.

The agent of the present invention can comprise any of components (A) to (D) as an active ingredient and it is preferable for the agent of the present invention to comprise component (A) as an active ingredient. The composition of the present invention may comprise two or more kinds selected from components (A) to (D) as active ingredients and it is preferable for the composition of the present invention to comprise at least component (A).

The first embodiment of the present invention includes an agent wherein component (A) is an active ingredient. Where component (A) is an active ingredient, such an agent can exhibit an effect of preventing and/or relieving osteoporosis.

The dose of the agent containing component (A) as an active ingredient is not particularly limited, provided that the effects of the present invention are not impaired, and can be changed, as needed, depending on various factors such as the age and the state of the subject living organism. For the desired effect of preventing and/or relieving osteoporosis to be obtained, the dose of the agent is preferably 0.001 g/day to 100 g/day as the amount of an ascorbic acid derivative to an adult.

The second embodiment of the present invention includes a composition containing component (A) and component (B) as active ingredients. By containing component (A) and component (B) as active ingredients, such a composition can be administered orally.

As for the composition according to the second embodiment of the present invention, the weight ratio of component (B) to component (A) is not particularly limited, provided that the effects of the present invention can be exhibited, and the ratio of component (B) to 1 part by weight of component (A) is usually 0.001 to 10,000 parts by weight, preferably 0.01 to 5,000 parts by weight, more preferably 0.1 to 3,000 parts by weight, and further preferably 0.1 to 2,000 parts by weight.

The composition according to the second embodiment of the present invention may comprise component (C). In this case, the total amount of component (B) and component (C) relative to 1 part by weight of component (A) is within the range of 0.001 to 10,000 parts by weight, preferably 0.01 to 5,000 parts by weight, more preferably 0.1 to 3,000 parts by weight, and further preferably 0.1 to 2,000 parts by weight.

The dose of the composition according to the second embodiment of the present invention is not particularly limited provided that the effects of the present invention are not impaired, and can be changed, as needed, depending on various factors such as the age and the state of the subject living organism. For the desired effect to be obtained, the dose is preferably 0.001 g/day to 100 g/day to an adult in terms of the amount of component (A) in the composition and is preferably 0.005 mg/day to 500 mg/day to an adult in terms of the amount of component (B) in the composition. When the composition comprises component (C), the dose to an adult is preferably 150 mg/day or more, and more preferably 300 to 450 mg/day in terms of the amount of component (C) in the composition.

In the composition according to the second embodiment of the present invention, by combining component (A) and component (B), component (B) can enhance the action of promoting bone formation of component (A). When component (A) and component (B) are further combined with component (C), the action of promoting bone formation is further enhanced and action of promoting cell growth is added. Accordingly, the present invention also provides the following inventions:
an agent for improving the bone formation-promoting function of an ascorbic acid derivative, the agent comprising an extract from a plant in the family Piperaceae as an active ingredient;
an agent for improving the bone formation-promoting function of a food or drink product that comprises an ascorbic acid derivative, the agent comprising an extract from a plant in the family Piperaceae as an active ingredient;
an agent for improving the bone formation-promoting function of a food or drink product that comprises an ascorbic acid derivative and lactoferrin, the agent comprising an extract from a plant in the family Piperaceae as an active ingredient;
a composition for improving the bone formation-promoting function of an ascorbic acid derivative, the composition comprising an extract from a plant in the family Piperaceae and lactoferrin as active ingredients;
a composition for improving the bone formation-promoting function of a food or drink product that comprises an ascorbic acid derivative, the composition comprising an extract from a plant in the family Piperaceae and lactoferrin as active ingredients.

The third embodiment of the present invention includes a composition comprising component (A) and component (D) as active ingredients. By comprising component (A) and component (D) as active ingredients, such a composition can exhibit action of promoting bone formation.

As for the composition according to the third embodiment of the present invention, the weight ratio of component (D) to component (A) is not particularly limited, provided that the effects of the present specification can be exhibited, and the ratio of component (D) to 1 part by weight of component (A) is usually 0.001 to 100,000 parts by weight, preferably 0.01 to 50000 parts by weight, more preferably 0.1 to 3,000 parts by weight, and further preferably 0.1 to 2,000 parts by weight.

The dose of the composition according to the third embodiment of the present invention is not particularly limited provided that the effects of the present invention are not impaired, and can be changed, as needed, depending on various factors such as the age and the state of the subject living organism. For the desired effect to be obtained, the dose is preferably 0.001 g/day to 100 g/day to an adult in terms of the amount of component (A) and is preferably 0.001 g/day to 10 g/day to an adult in terms of the amount of component (D).

In the composition, by combining component (A) and component (D), the action of promoting bone formation is exhibited, and therefore the composition can also be called a composition for promoting bone formation. In the present invention, when component (A) is combined with component (D), component (D) can enhance the action of promoting bone formation of component (A). Therefore, the present invention also provides an agent comprising sorbitol and/or a derivative thereof as an active ingredient for improving the bone formation-promoting function of an ascorbic acid derivative, and an agent comprising sorbitol and/or a derivative thereof as an active ingredient for improving the bone formation-promoting function of a food or drink product that comprises an ascorbic acid derivative.

The agent of the present invention can be used as it is as a final product such as a food or drink product, a pharmaceutical product, or a quasi-pharmaceutical product. Alternatively, the agent of the present invention can be used as an additive for a food or drink product, an additive for a pharmaceutical product, or an additive for a quasi-pharmaceutical product. In this way, food or drink products, pharmaceutical products, and quasi-pharmaceutical products can be imparted with an effect of preventing and/or relieving osteoporosis and/or action such as the action of promoting bone formation. Alternatively, the agent of the present invention may comprise a component (a pharmacologically acceptable base) other than active ingredients so as to be used as a composition. The composition may comprise a component (a pharmacologically acceptable base) other than active ingredients. The composition can be used as it is as a final product such as a food or drink product, a pharmaceutical product, or a quasi-pharmaceutical product. The composition may be used as an additive for a food or drink product, an additive for a pharmaceutical product, or an additive for a quasi-pharmaceutical product. In this way, food and drink products, pharmaceutical products, and quasi-pharmaceutical products can be imparted with an effect of preventing and/or relieving osteoporosis and/or action such as the action of promoting bone formation.

The composition of the present invention can be used as it is as a final product such as a food or drink product, a pharmaceutical product, or a quasi-pharmaceutical products. Alternatively, the composition of the present invention can be used as an additive for a food or drink product, an additive for a pharmaceutical product, or an additive for a quasi-pharmaceutical product. In this way, food and drink products, pharmaceutical products, and quasi-drugs can be imparted with an effect of preventing and/or relieving osteoporosis.

The composition of the present invention may comprise a component (a pharmacologically acceptable base) other than active ingredients. Examples of the other component may include components such as a storage stabilizer for ensuring stability primarily during storage and distribution. In addition, one or more components (preferably about one to three components and more preferably about one component) selected from various components that constitute the desired final product such as a food or drink product, a pharmaceutical product, or a quasi-pharmaceutical product may also be comprised.

Examples of the component other than active ingredients may include components such as a storage stabilizer for ensuring stability primarily during storage and distribution. In addition, one or more components (preferably about one to three components and more preferably about one component) selected from various components that constitute the desired final product such as a food or drink product, a pharmaceutical product, or a quasi-pharmaceutical product may also be comprised.

The component other than active ingredients is not particularly limited provided that the objects of the present invention are not impaired. For example, one, two, or more components can be selected from excipients, disintegrating agents, binding agents, lubricants, coating agents, colorants, color formers, taste masking agents, flavoring agents, antioxidants, antiseptics, taste ingredients, acidulants, sweeteners, fortifiers, vitamin compounds, inflating agents, thickeners, surfactants, and the like provided that the components do not impair essential properties such as formulation stability of a formulation and suit the dosage form of a final product such as a pharmaceutical product, a quasi-pharmaceutical product, or a food or drink product.

A component other than component (A) which can be combined with the agent according to the first embodiment of the present invention may be another component having an effect of preventing and/or relieving osteoporosis. A component other than components (A) to (C) which can be comprised in the composition according to the second embodiment of the present invention may be another component having an effect of promoting bone formation. A component other than components (A) and (D) which can be comprised in the composition according to the third embodiment of the present invention may be another component having an effect of promoting bone formation.

When the composition of the present invention comprises component (A), the formulated amount of component (A) is not particularly limited provided that the effects of the present invention can be exhibited, and is usually 0.00001% or more by mass, preferably 0.001% or more by mass, more preferably 0.01% or more by mass, further preferably 0.1% or more by mass, and particularly preferably 1% or more by mass relative to the whole composition. The upper limit to the formulated amount is preferably 35% or less by mass, more preferably 25% or less by mass, further preferably 20% or less by mass, and particularly preferably 15% or less by mass relative to the whole composition.

When the composition of the present invention comprises component (C), the formulated amount of component (C) is not particularly limited provided that the effects of the present invention can be exhibited, and is selected, as needed, depending on the dosage form, the form of administration, and the subject. Usually for oral administration, the formulated amount of component (C) is preferably 0.01% to 10% (% by mass of solid matter) and is more preferably 0.1% to 4% relative to the whole composition. The upper limit to the formulated amount is preferably 10% or less (% by mass of solid matter) and is more preferably 4% or less relative to the bone forming composition as a whole.

The form of administration of the composition of the present invention is not particularly limited. Examples thereof may include oral administration such as buccal administration, sublingual administration and parenteral administration such as intravenous administration, intramuscular administration, subcutaneous administration, transdermal administration, transnasal administration, and pulmonary administration. Among these, less-invasive forms of administration are preferable, oral administration is more preferable, and oral administration in the form of a food or drink product is further preferable.

The dosage form of the composition of the present invention is not particularly limited and can be determined, as needed, depending on whether the composition is made into a food or drink product, a pharmaceutical product, or a quasi-pharmaceutical product. Examples of the dosage form for oral administration may include liquid forms (liquid agents), syrup forms (syrup agents), tablet forms (pills, tablets), capsule forms (capsules), powder forms (granules, fine particles), soft capsule forms (soft capsules), syrup forms (syrup agents), solid forms, semiliquid forms, cream forms, and paste forms.

The composition according to the second embodiment of the present invention is preferably an enteric coated agent. In this case, an enteric component is preferably formulated therein as an optional component. Examples of the enteric component may include shellac, hydroxymethyl cellulose phthalate, carboxymethylcellulose, cellulose acetate phthalate, methacrylic acid copolymers, ethylcellulose, aminoalkyl methacrylate copolymers, brewer's yeast cell wall such as a product with a trade name YeastWrap, tapioca starch, gelatin, and pectin. Whether the agent is enteric can be confirmed by The Japanese Pharmacopoeia Fourteenth Edition, Disintegration Test.

The method for producing the composition of the present invention is not particularly limited and is selected, as needed, depending on the dosage form and/or the like. An example thereof is a method to mix all the active ingredients and optional components and then compression molding the resulting mixture to prepare a tablet. Additionally in this method, the resulting tablet is preferably coated with an enteric component such as shellac.

The method to take the composition of the present invention varies depending on the dosage form and is not particularly limited. When the dosage form is a tablet, for example, the tablet is preferably taken with water and/or the like.

The timing of administration of the composition of the present invention is not particularly limited. The interval between a meal and administration is not particularly limited either, and administration can be performed before, after, or between meals. Administration may be performed before the development of osteoporosis or may be performed after the development of osteoporosis. In the case of administration after the development of osteoporosis, the administration is preferably performed in an early stage of the development.

The agent according to the first embodiment of the present invention and a composition containing the same can prevent the development of osteoporosis when administered before the development, while when administered after the development of osteoporosis, the symptom can be relieved (alleviated). Because of this, the agent and a composition comprising the same can be used as a food or drink product or a pharmaceutical product for preventing and/or relieving osteoporosis.

Osteoporosis is broadly classified into primary osteoporosis and secondary osteoporosis according to the cause. The present invention can exhibit the preventing and relieving effects on either type of osteoporosis regardless of the cause of osteoporosis. Examples of primary osteoporosis may include postmenopausal osteoporosis, senile osteoporosis, and osteoporosis accompanied by pregnancy. Examples of secondary osteoporosis may include osteoporosis with an underlying disease such as diabetes.

The agent according to the first embodiment of the present invention and a composition comprising the same can also exhibit an effect of preventing fractures. The cause, the site, the severity, and the like of fractures are not particularly limited, and fractures caused by osteoporosis are the primary subject.

The composition according to the second embodiment of the present invention can exhibit an effect of promoting bone formation and, in other words, can enhance the activity of genes such as collagen gene and alkaline phosphatase gene in bone tissue to promote the production of bone tissue. Because of this, the composition according to the second embodiment of the present invention can also be called a composition for promoting bone formation. When the composition according to the second embodiment of the present invention is administered before the development of osteoporosis, the development can be prevented. When the composition according to the second embodiment of the present invention is administered after the development of osteoporosis, the symptom can be relieved (alleviated). Because of this, the composition according to the second embodiment of the present invention can also be called a composition for preventing and/or relieving osteoporosis. The composition according to the second embodiment of the present invention can be used as a food or drink product or a pharmaceutical product for preventing and/or relieving osteoporosis.

The composition according to the second embodiment of the present invention can also exhibit an effect of preventing fractures and therefore can also be called a composition for preventing fractures. The cause, the site, the severity, and the like of fractures are not particularly limited, and fractures caused by osteoporosis are the primary subject.

The composition according to the third embodiment of the present invention can exhibit an effect of promoting bone formation and, in other words, can enhance the activity of genes such as collagen gene and alkaline phosphatase gene in bone tissue to promote the production of bone tissue. When the third composition of the present invention is administered before the development of osteoporosis, the development can be prevented. When the composition according to the third embodiment of the present invention is administered after the development of osteoporosis, the symptom can be relieved (alleviated). Because of this, the third composition of the present invention can also be called a composition for preventing and/or relieving osteoporosis. The composition according to the third embodiment of the present invention can be used as a food or drink product or a pharmaceutical product for preventing and/or relieving osteoporosis.

The composition according to the third embodiment of the present invention can also exhibit an effect of preventing fractures and therefore can also be called a composition for preventing fractures. The cause, the site, the severity, and the like of fractures are not particularly limited, and fractures caused by osteoporosis are the primary subject.

The agent according to the first embodiment of the present invention and a composition comprising the same can also be used as an agent for reducing fracture risk, an agent for increasing bone mass, an agent for increasing a bone density, and an agent for enhancing bone strength.

With the effect of promoting bone formation, the composition according to the second embodiment of the present invention can also be used as a composition for increasing bone mass, a composition for increasing a bone density, and a composition for enhancing bone strength.

With the effect of promoting bone formation, the composition according to the third embodiment of the present invention can also be used as a composition for increasing bone mass, a composition for increasing a bone density, and a composition for enhancing bone strength.

The subject to take the agent and the composition of the present invention is not particularly limited, and examples thereof may include subjects already having osteoporosis and subjects with the risk of developing osteoporosis and fractures such as the elderly, smokers, pregnant women, and postmenopausal women. Even subjects with no particular problem can take the agent and the composition of the present invention routinely for preventing osteoporosis or fractures, for increasing bone mass, for increasing a bone density, and for enhancing bone strength.

The agent and the composition of the present invention can be used in various food and drink products. Examples thereof may include: beverages such as soft drinks, carbonated beverages, energy drinks, powdered drinks, fruit beverages, milk drinks, and jelly drinks; confectionery such as cookies, cakes, chewing gums, candies, tablets, gummy candies, steamed buns with filing (*manju*)*,* adzuki bean jellies (*yokan*), puddings, jellies, ice creams, and sherbets; processed marine products such as boiled fish paste (*kamaboko*), baked tubular rolls of fish pastes (*chikuwa*), and boiled cakes made of ground fish (*hampen*); processed livestock products such as hamburgers, hams, sausages, wiener sausages, butter, yogurt, fresh cream, cheese, and margarine; soup such as powdered soups, and liquid soups; staple food such as rice, noodles including dried noodles and fresh noodles, bread, and cereals; and seasonings such as mayonnaise, shortening, dressings, sauces, dipping sauces (tare), and soy sauce. The agent and the composition of the present invention can also be used in food and drink products such as health foods, functional foods, dietary supplements (supplements), nutrient supplements, foods for specified health uses, foods for medical use, foods for the sick, baby foods, foods for use in nursing care, and foods for the elderly. Among them, use in dietary supplements is preferable and use in dietary supplements in a tablet form is more preferable.

### Examples

### Examples 1 and 2 and Comparative Examples 1 and 2

Type 2 diabetic model mice (KKAy mice (8-week-old, male)) were divided into three test groups, and the mice in each test group were administered with 1% (W/V) ascorbic acid phosphate Mg salt (APM) (Example 1) (n=8), 1% (W/V) ascorbic acid 2-O-α-glucoside (A2G) (Example 2) (n=9), or distilled water (DW) (Comparative Example 1) (n=9). Administration was performed in a way of freely accessible drinking water. Duration of administration was 14 weeks (discontinued when the mice turned 22 weeks old).

On the other hand, healthy mice (C57BL/6 mice (8-week-old, male)) were administered with distilled water (DW) (Comparative Example 2) (n=9). The method of administration and duration of administration were the same as for the type 2 diabetic model mice.

APM used was a commercially available product manufactured by Showa Denko K.K. A2G used was a commercially available product manufactured by Hayashibara Biochemical Laboratories, Inc.

### [Evaluation of increase in a bone density]

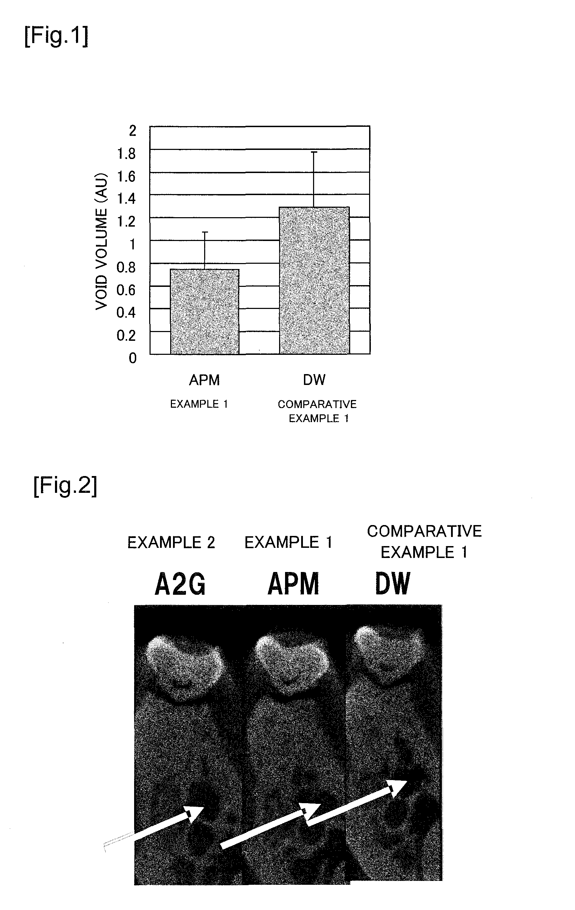

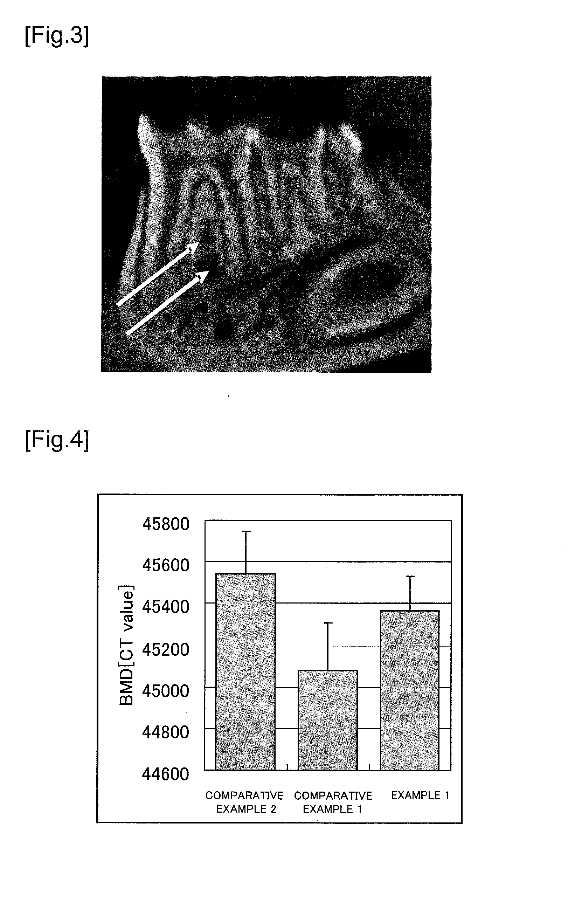

At the end of duration of administration, each mouse was subjected to X-ray CT for tomographic images of cancellous bone between the roots of the lower first molar, followed by measurement of the volume of voids (the areas of voids in the successive tomographic images were integrated). The measured values were used to calculate the average value for each test group. The results of the volume (unit: AU (arbitrary unit)) of voids in cancellous bone in Example 1 (APM) and Comparative Example 1 (DW) are depicted in FIG. 1. The sections of cancellous bone in Example 1 (APM), Example 2 (A2G), and Comparative Example 1 (DW) are depicted in FIG. 2. The section of cancellous bone in Comparative Example 2 (healthy mouse) is depicted in FIG. 3. In FIG. 2 and FIG. 3, the parts pointed by arrows are voids in the cancellous bone.

In the type 2 diabetic model mice, voids were observed in the cancellous bone. However, in the type 2 diabetic model mice to which APM was administered, the volume of voids in cancellous bone was smaller than the volume of voids in cancellous bone in the type 2 diabetic model mice to which distilled water was administered (FIG. 1). Also in the type 2 diabetic model mice to which A2G was administered, as in the type 2 diabetic model mice to which APM was administered, a decrease was observed in the volume of voids in cancellous bone compared to that in the healthy mice (FIG. 2 and FIG. 3).

From tomographic images of cancellous bone between the roots taken in the same manner as above, a bone mineral density (BMD) was calculated. The resulting BMD values were compared in terms of the CT values of bone portions excluding the voids (the average value for mice in each test group). The BMD values in Example 1 (APM), Comparative Example 1 (DW), and Comparative Example 2 (healthy mice) are depicted in FIG. 4.

The BMD values of cancellous bone were lowered in the type 2 diabetic model mice (Comparative Examples 1 and 2). The BMD values in the type 2 diabetic model mice to which APM was administered (Example 1) were higher than the BMD values in the type 2 diabetic model mice to which distilled water was administered (Comparative Example 1) (FIG. 4).

These results indicate that by an ascorbic acid derivative, the balance between bone formation and bone resorption of cancellous bone is progressively shifted toward bone formation and the bone density (the ratio of bone/void) is increased. The results also indicate that an ascorbic acid derivative improves the BMD of the formed bone thereby enhancing the strength of the bone. The results further indicate that an ascorbic acid derivative has the potential to prevent osteoporosis and fractures, reduce the risk of these, and relieve symptoms of osteoporosis.

Examples 3 to 5, Comparative Examples 3 to 6, and Reference Example 1 (test on promotion of osteoblast differentiation (promotion of bone formation))

The activity of the second composition of the present invention to promote bone formation was evaluated from the expression level of alkaline phosphatase (ALP) gene in an osteoblast culture system.

MG-63 cells (DS Pharma Biomedical Co., Ltd.), which are human osteosarcoma-derived osteoblasts, were cultured in an EMEM culture solution containing 10% FBS, 1% non-essential amino acid, and 2 mM glutamine at 37°C in 5%CO₂-95%Air to achieve confluency. Trypsin treatment was performed to collect the cells, which were then inoculated in the same culture solution as above in a 6-well plate to achieve 1 × 10⁵ cells/9.6 cm², followed by culture for another 6 hours for adhesion of the cells to the plate. Subsequently, the culture solution was replaced by a calcification medium, followed by culture for another 21 days. The calcification medium was prepared by adding 10-mmol/L of β-glycerophosphoric acid and components depicted in Table 1 to the above culture solution. The amount of Piper longum L. extract, when added, was 0.01% (100 ppm). Cells cultured in medium alone served as a reference example.

After the culture supernatant was removed and the cells were rinsed with a phosphate buffer at pH7.4, RNA was extracted with RNeasy mini kit from QIAGEN, followed by real-time PCR to determine the expression level of alkaline phosphatase (ALP) mRNA, whereby the ratio of expression of ALP gene to that of HPRT (hypoxanthine-guanine phosphoribosyltransferase: housekeeping gene) gene was calculated.

For each example, with expression when sodium L-ascorbate alone was added (Comparative Example 4) being defined as 1, relative expression was calculated. The evaluation criteria depicted in Table 2 were applied to the resulting relative expression level to evaluate expression of ALP gene.

As described above, the ascorbic acid derivative was added to the calcification medium to achieve 250 µM in terms of ascorbic acid, specifically, 50 µg/mL of sodium L-ascorbate (AA), 99.4 µg/mL of L-ascorbic acid phosphate magnesium salt (APM), and 85.4 µg/mL of L-ascorbic acid 2-O-α-glucoside (A2G).

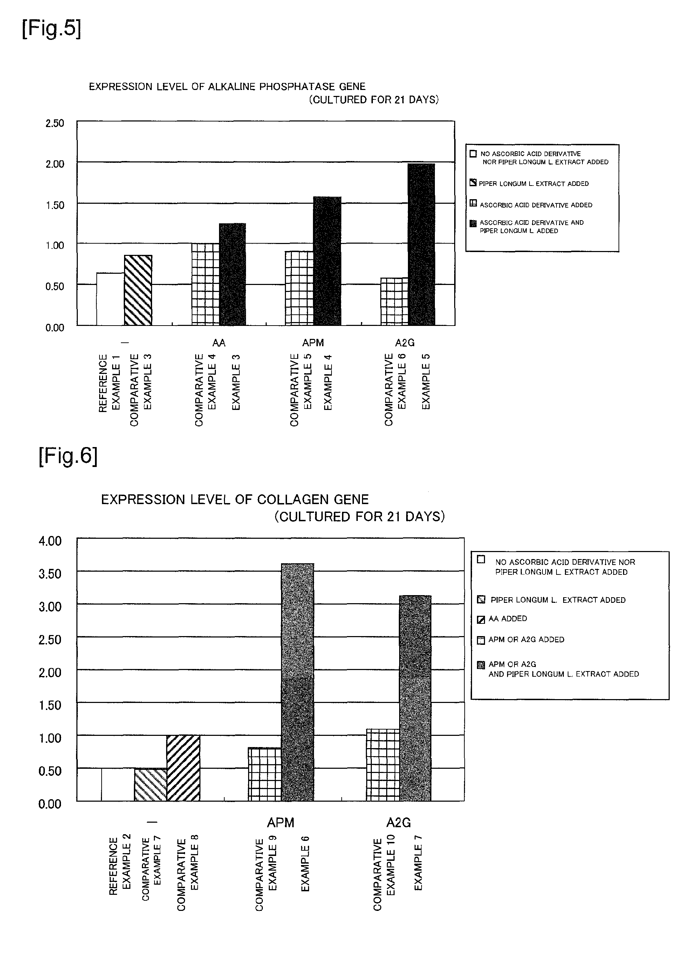

AA used was a commercially available reagent (trade name: L(+)-Ascorbic Acid Sodium Salt) from Wako Pure Chemical Industries, Ltd. APM used was a commercially available product manufactured by Showa Denko K.K. A2G used was a commercially available product manufactured by Hayashibara Biochemical Laboratories, Inc.. The extract from a plant in the family Piperaceae used was Piper longum extract MF from Maruzen Pharmaceuticals Co., Ltd. The results are listed in Table 1 and FIG. 5.

[Table 1]

**Table 1**

| | | Component (A): ascorbic acid derivative | Component (B): extract from plant in family Piperaceae | Evaluation |
|---|---|---|---|---|
| Reference Example | 1 | - | - | - |
| Comparative Example | 3 | - | Piper longum L. extract | Δ |
| | 4 | AA | - | Δ |
| | 5 | APM | - | Δ |
| | 6 | A2G | - | × |
| Example | 3 | AA | Piper longum L. extract | ○ |
| | 4 | APM | Piper longum L. extract | ○ |
| | 5 | A2G | Piper longum L. extract | ○○ |

[Table 2]

**Table 2**

| Criteria | Gene expression | | |
|---|---|---|---|
| ×: | 0≤ | to | <0.8 |
| Δ: | 0.8≤ | to | <1.2 |
| ○: | 1.2≤ | to | <1.8 |
| ○○: | 1.8≤ | to | <2.5 |
| ○○○: | 2.5≤ | | |

As is obvious from Table 1 and FIG. 5, high activity of ALP gene expression was observed in Examples 3 to 5 compared to Comparative Examples 3 to 6. From evaluation of ALP gene expression in Examples 3 to 5 on the criteria in Table 2, Example 5 was rated as ○○. These results indicates that the compositions conprising component (A) and (B) as active ingredients exhibites an excellent effect of promoting bone formation and that using an ascorbic acid derivative as component (A) resultes in an even more significant effect of promoting bone formation. It is also indicated that the second composition of the present invention promotes bone formation to possibly increase the bone mass and eventually give an effect of preventing and relieving osteoporosis.

Examples 6 and 7, Comparative Examples 7 to 10, and Reference Example 2 (test on promotion of osteoblast differentiation (promotion of bone formation))

The activity of the second composition of the present invention to promote bone formation was evaluated from the expression level of type I collagen gene in an osteoblast culture system.

Cell culture in Examples 6 and 7 were performed in the same manner as in Examples 4 and 5, respectively.

Cell culture in Comparative Examples 7 to 10 were performed in the same manner as in Comparative Examples 3 to 6, respectively.

After the culture supernatant was removed and the cells were rinsed with a phosphate buffer at pH7.4, RNA was extracted with RNeasy mini kit from QIAGEN, followed by real-time PCR to determine the expression level of type I collagen mRNA, whereby the ratio of expression of type I collagen gene to that of HPRT gene was calculated.

For each of Examples 6 and 7 and Comparative Examples 7 to 10, with expression when an ascorbic acid derivative (AA) was added (Comparative Example 8) being defined as 1, relative expression was calculated. The evaluation criteria listed in Table 2 were applied to the resulting relative expression level to evaluate expression of type I collagen gene. The results in Examples 6 and 7 and Comparative Examples 7 to 10 are listed in Table 3 and FIG. 6.

[Table 3]

**Table 3**

| | | Component (A): ascorbic acid derivative | Component (B): extract from plant in family Piperaceae | Evaluation |
|---|---|---|---|---|
| Reference Example | 2 | - | - | - |
| Comparative Example | 7 | - | Piper longum L. extract | × |
| | 8 | AA | - | Δ |
| | 9 | APM | - | Δ |
| | 10 | A2G | - | Δ |
| Example | 6 | AA | Piper longum L. extract | ○○○ |
| | 7 | APM | Piper longum L. extract | ○○○ |

As is obvious from Table 3 and FIG. 6, the high gene expression levels were observed in Examples 6 and 7 compared to Comparative Examples 7 to 10. From evaluation of expression of type I collagen gene in Examples 6 and 7 on the criteria in Table 2, both of Examples 6 and 7 were rated as ○○○. These results indicates that the composition of the present invention exhibits an excellent effect of promoting bone formation and promotes bone formation to possibly increase the bone mass and eventually give an effect of preventing and relieving osteoporosis.

Examples 8 to 11, Comparative Examples 11 to 15, and Reference Example 3 (test on promotion of osteoblast differentiation (promotion of bone formation))

The activity of the second composition of the present invention to promote bone formation was evaluated in the same manner as in Example 6 from the expression level of type I collagen gene in an osteoblast culture system.

Cell culture in Examples 8 and 9 were performed in the same manner as in Examples 6 and 7, respectively, except that duration of culture was 14 days.

Cell culture in Examples 10 and 11 were performed in the same manner as in Examples 6 and 7, respectively, except that lactoferrin was further added and duration of culture was 14 days. Lactoferrin was added at 300 ppm. Lactoferrin used was bovine lactoferrin (bLF) manufactured by DMW.

Cell culture in Comparative Examples 11 and 13 to 15 were performed in the same manner as in Comparative Examples 7 to 10, respectively, except that duration of culture was 14 days.

Cell culture in Comparative Example 12 was performed in the same manner as in Comparative Example 7 except that lactoferrin was further added and duration of culture was 14 days. Lactoferrin was added at 300 ppm. Lactoferrin used was bovine lactoferrin (bLF) manufactured by DMW.

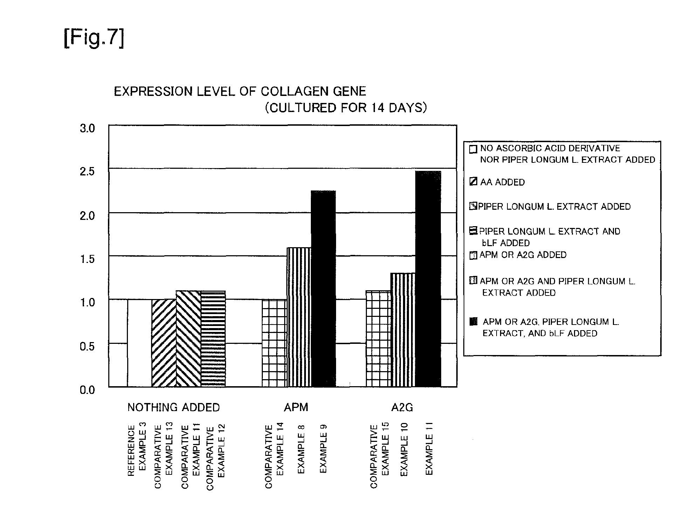

The ratio of expression of collagen gene to that of HPRT gene was calculated in the same manner as in Example 6. For each example and comparative example, with expression when an ascorbic acid derivative (AA) was added (Comparative Example 13) being defined as 1, relative expression was calculated. The evaluation criteria listed in Table 2 were applied to the resulting relative expression level to evaluate expression of collagen gene. The results in Examples 8 to 11 and Comparative Examples 11 to 15 are listed in Table 4 and FIG. 7.

[Table 4]

**Table 4**

| | | Component (A): ascorbic acid derivative | Component (B): extract from plant in family Piperaceae | Component (C): lactoferrin | Evaluation |
|---|---|---|---|---|---|
| Reference Example | 3 | - | - | - | - |
| Comparative Example | 11 | - | Piper longum L. extract | - | × |
| | 12 | - | Piper longum L. extract | bLF | × |
| | 13 | AA | - | - | Δ |
| | 14 | APM | - | - | Δ |
| | 15 | A2G | - | - | Δ |
| Example | 8 | APM | Piper longum L. extract | - | ○ |
| | 9 | A2G | Piper longum L. extract | - | ○ |
| | 10 | APM | Piper longum L. extract | bLF | ○○ |
| | 11 | A2G | Piper longum L. extract | bLF | ○○○ |

As is obvious from Table 4 and FIG. 7, high gene expression levels were observed for the combination of an ascorbic acid derivative and Piper longum L. extract in Examples 8 and 9 compared to Comparative Examples 11 to 15, and further addition of lactoferrin in Examples 10 and 11 resulted in an even more significant gene expression level compared to Comparative Examples 11 to 15. From evaluation of expression of collagen gene in Examples 8 to 11 on the criteria in Table 2, Example 10 was rated as ○○ and Example 11 was rated as ○○○. These results indicates that the second composition of the present invention exhibits an excellent effect of promoting bone formation and promotes bone formation to possibly increase the bone mass and eventually give an effect of preventing and relieving osteoporosis.

Examples 10 and 11 (duration of culture: 14 days) gave excellent results, which were comparable to the results in examples with duration of culture of 21 days listed in Table 1 and Table 2. These results indicates that the second composition of the present invention gives even better results when containing component (C) and that an effect of promoting bone formation is possibly exhibited even in a short period of time.

Examples 12 to 14 and Comparative Examples 16 to 19 (test on promotion of osteoblast differentiation (promotion of bone formation))

The activity of the third agent of the present invention to promote bone formation was evaluated from the expression level of alkaline phosphatase (ALP) gene in an osteoblast culture system.

MG-63 cells (DS Pharma Biomedical Co., Ltd.), which were human osteosarcoma-derived osteoblasts, were cultured in an EMEM culture solution containing 10% FBS, 1% non-essential amino acid, and 2 mM glutamine at 37°C in 5%CO₂-95%Air to achieve confluency. Trypsin treatment was performed to collect the cells, which were then inoculated in the same culture solution as above in a 6-well plate to achieve 1 × 10⁵ cells/9.6 cm², followed by culture for another 6 hours for adhesion of the cells to the plate. Subsequently, the culture solution was replaced by a calcification medium, followed by culture. The duration of culture was 21 days. The calcification medium was prepared by adding 10-mmol/L of β-glycerophosphoric acid and components listed in Table 5 to the above culture solution. The amount of sorbitol, when added, was 20,000 ppm.

After the culture supernatant was removed and the cells were rinsed with a phosphate buffer at pH7.4, RNA was extracted with RNeasy mini kit from QIAGEN, followed by calculation of the ratio of expression of alkaline phosphatase gene to that of HPRT (hypoxanthine-guanine phosphoribosyltransferase: housekeeping gene).

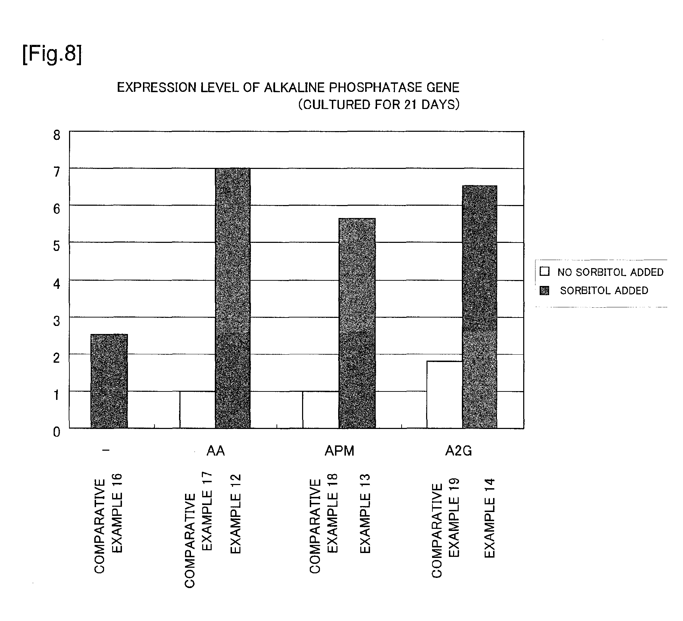

For each example and comparative example, with expression when sodium ascorbate (AA) was added (Comparative Example 1) being defined as 1, relative expression was calculated. The results are depicted in FIG. 8.

The ascorbic acid derivative was added to achieve 250 µM in terms of ascorbic acid, specifically, 50 µg/mL of sodium L-ascorbate (AA), 99.4 µg/mL of L-ascorbic acid phosphate magnesium salt (APM), and 85.4 µg/mL of L-ascorbic acid 2-0-α-glucoside (A2G).

AA used was a commercially available reagent (trade name: L(+)-Ascorbic Acid Sodium Salt) from Wako Pure Chemical Industries, Ltd. APM used was a commercially available product manufactured by Showa Denko K.K. A2G used was a commercially available product manufactured by Hayashibara Biochemical Laboratories, Inc. Sorbitol used was a commercially available product (trade name: D-sorbitol) from Wako Pure Chemical Industries, Ltd.

[Table 5]

**Table 5**

| | Component (A) | Component (D) |
|---|---|---|
| Comparative Example 16 | AA | - |
| Comparative Example 17 | APM | - |
| Comparative Example 18 | A2G | - |
| Comparative Example 19 | - | Sorbitol |
| Example 12 | AA | Sorbitol |
| Example 13 | APM | Sorbitol |
| Example 14 | A2G | Sorbitol |

As is obvious from FIG. 8, high gene expression levels were observed in Examples 12 to 14 compared to Comparative Examples 16 to 19. These results indicate that the third agent of the present invention exhibits an excellent effect of promoting bone formation and promotes bone formation to possibly increase the bone mass and eventually give an effect of preventing and relieving osteoporosis.

## Claims

1. An agent for preventing and/or relieving osteoporosis, the agent comprising component (A): an ascorbic acid derivative as an active ingredient.

2. The agent according to claim 1, wherein component (A) is ascorbic acid phosphate magnesium salt and/or ascorbic acid 2-glucoside.

3. A composition for preventing and/or relieving osteoporosis, the composition comprising component (A): an ascorbic acid derivative and a pharmacologically acceptable carrier.

4. A composition for oral administration, comprising component (A): an ascorbic acid derivative and component (B): an extract from a plant in the family Piperaceae as active ingredients.

5. The composition according to claim 4, wherein component (B) is an extract of Piper longum L.

6. The composition according to claim 4 or 5, wherein component (A) is one or more ascorbic acid derivatives selected from sodium ascorbate, ascorbic acid phosphate magnesium salt, and ascorbic acid 2-glucoside.

7. The composition according to any one of claims 4 to 6, further comprising component (C): lactoferrin.

8. The composition according to claim 7, wherein component (C) is lactoferrin derived from cow milk.

9. The composition according to any one of claims 4 to 8, wherein the composition promotes bone formation or prevents and/or relieves osteoporosis.

10. A composition for promoting bone formation, the composition comprising component (A): an ascorbic acid derivative and component (D): sorbitol and/or a sorbitol derivative as active ingredients.

11. The composition according to claim 10, wherein the composition prevents and/or relieves osteoporosis.

12. The composition according to any one of claims 3 to 11, further comprising a pharmacologically acceptable carrier.

13. The composition according to any one of claims 3 to 12, wherein the composition is a pharmaceutical product, a quasi-pharmaceutical product, or a food or drink product.
